# EUROPÄISCHE PATENTANMELDUNG

(11) **EP 2 481 757 A1**
(43) Veröffentlichungstag der Anmeldung: **01.08.2012**
(21) Anmeldenummer: 12165439.6
(22) Anmeldetag: 02.04.2007
(51) Int. Cl.: C07K 16/28, A61K 39/395, A61P 19/02, A61P 25/28, A61P 29/00, A61P 37/00

(54) **Behandlung von multipler Sklerose und/oder rheumatoider Arthritis**

(30) Priorität: 03.04.2006 DE 102006015341
(62) Teilanmeldung aus: 07723870.7
(71) Anmelder: Georg-August-Universität Göttingen Stiftung Öffentlichen Rechts Universitätsmedizin, 37099 Göttingen (DE); Klinikum der Universität Regensburg - Anstalt des Öffentlichen Rechts-, 93053 Regensburg (DE)
(72) Erfinder: Prinz, Marco, 79100 Freiburg (DE); Brück, Wolfgang, 37124 Rosdorf (DE); Mildner, Alexander, 30966 Hemmingen (DE); Mack, Matthias, 93051 Regensburg (DE)
(74) Vertreter: Wichmann, Hendrik

(57) **Zusammenfassung**

Die Erfindung betrifft die Verwendung eines Antikörpers, der an Loop 1 und Loop 2 des Chemokinrezeptors CCR2 spezifisch binden kann, zur Herstellung eines Medikamentes zur Behandlung von multipler Sklerose und/oder rheumatoider Arthritis bei einem Subjekt. Vorzugsweise ist das Subjekt ein Primat oder Mensch. In einer anderen Ausführungsform betrifft die vorliegende Erfindung die Verwendung eines Antikörpers, der an Loop 1 und Loop 2 des Chemokinrezeptors CCR2 spezifisch binden kann, in einem Verfahren zum Depletieren von Monozyten.

## Beschreibung

Die vorliegende Erfindung betrifft die Behandlung von multipler Sklerose und/oder rheumatoider Arthritis mit Hilfe von Antikörpern.

Multiple Sklerose ist eine Erkrankung des zentralen Nervensystems, bei der die Markscheiden der Nervenfasern zerstört werden, d.h. die Nervenfasern werden demyelinisiert. Ursache und Pathophysiologie sind bisher nur schlecht verstanden. Vermutlich handelt es sich um einen exogen und endogen modulierten immunologischen Prozess. Die Symptome der multiplen Sklerose sind relativ unspezifisch und umfassen beispielsweise spastische Lähmungen und andere motorische und sensible Ausfälle.

Eine befriedigende Therapie der multiplen Sklerose ist bisher nicht möglich. Beispielsweise erfolgt eine Therapie bisher durch hochdosierte Verabreichung von Steroiden, sowie durch Verabreichung von Azathrioprin, Glatirameracetat, Methotrexat, Mitoxantron oder durch immunmodulierende Behandlung, z.B. mit Interferonen. Dies führt jedoch lediglich zu einer Verringerung der Frequenz und Intensität der Krankheitsschübe. Im Übrigen orientiert sich die Behandlung an der Linderung der Symptome.

Schneider et al. erwähnen, dass die Depletion von Zellen, die den Chemokinrezeptor CCR2 exprimieren, eine mögliche Strategie zur Behandlung von inflammatorischen Erkrankungen darstellen könnte (Schneider, M.A., Brühl, H. Wechselberger, A., et al. (2005), In vitro and in vivo properties of a dimeric bispecific single-chain antibody IgGfusion protein for depletion of CCR2+ target cells in mice. European Journal of Immunology, Bd. 35, S. 987-995). Die Autoren beschreiben die Lyse von CCR2-positiven Zellen in Mäusen mit Hilfe eines bispezifischen Antikörpers, der unter anderem gegen CCR2 gerichtet ist. Es wird jedoch keinerlei therapeutische Wirkung gezeigt.

Rheumatoide Arthritis ist eine chronisch entzündliche Systemerkrankung, die vorwiegend die Gelenke befällt und zu Deformierungen sowie Bewegungseinschränkungen führt. Symptome sind zum Beispiel Schwellung, Schmerz und Bewegungseinschränkung. Die Ursache der rheumatoiden Arthritis ist noch ungeklärt, diskutiert werden unter anderem eine Immunpathogenese sowie infektiöse Ursachen und genetische Faktoren.

Die Therapie der rheumatoiden Arthritis erfolgt typischerweise mit so genannten Antirheumatika, d.h. z.B. mit DMARDs (disease modifying antirheumatic drugs), zu denen beispielsweise Methotrexat und Leflunomid gehören. Zusätzlich können Steroide zum Einsatz kommen. Ferner können Biologika oder Immunsuppressiva, wie z.B. TNF-alpha Blocker (z.B. monoklonale Antikörper gegen TNF-alpha oder Ethernacept) oder IL-1 Rezeptor-Antagonisten (z.B. Anakinra) eingesetzt werden. Nichtsteroidale Antirheumatika (NSAR) werden vor allem zur Schmerztherapie eingesetzt. Dennoch gelingt es in vielen Fällen nicht, die Erkrankung befriedigend zu behandeln und Gelenkschäden, die bis zur Immobilisation gehen, zu vermeiden.

Brühl et al. beschreiben die Verabreichung von monoklonalen Antikörpern (MC-21), die gegen den Chemokinrezeptor CCR2 gerichtet sind, im Mausmodell der Kollagen-induzierten Arthritis (Bruhl H, Cihak J, Schneider MA, Plachy J, Rupp T, Wenzel I, Shakarami M, Milz S, Ellwart JW, Stangassinger M, Schlondorff D, Mack M. (2004) Dual role of CCR2 during initiation and progression of collagen-induced arthritis: evidence for regulatory activity of CCR2+ T cells. J Immunol., Bd. 172(2), S. 890-898). Sie beschreiben, dass eine Linderung der Arthritis durch die Antikörper nur erreicht wurde, wenn die Blockade von CCR2 während der Initiationsphase der Erkrankung (das bedeutet direkt nach der Immunisierung mit Kollagen und vor dem Auftreten der Gelenkentzündung) erfolgte, nicht jedoch während der Progressionsphase. Während der Progressionsphase bewirkte die Blockade von CCR2 sogar eine Verschärfung der Arthritis und der Immunantwort gegen Kollagen. Daraus ist zu schließen, dass durch die Antikörper lediglich die künstliche Erzeugung der Erkrankung in dem Modellsystem beeinflusst wurde, also die Immunisierung des Tieres mit Kollagen. Die Behandlung der Erkrankung selbst erscheint auf diese Weise jedoch nicht möglich.

Brodmerkel et al. (2005) beschreiben einen kleinmolekularen Antagonisten, INCB3344, des Maus CCR2 Rezeptors (Brodmerkel, C.M., Huber, R. Covington, M., Diamond, S., Hall, L., et al. (2005). Discovery and Pharmacological Characterization of a Novel Rodent-Active CCR2 Antagonist, INCB3344). Das Molekül wurde im Mausmodell der experimentellen autoimmunen Enzephalitis (EAE) studiert, einem Modell der multiplen Sklerose. Die Verabreichung begann allerdings bereits zu einem Zeitpunkt (0 Tage bzw. 7 Tage nach Immunisierung), zu dem nur wenige Tiere klinische Zeichen der Erkrankung zeigten. Brodmerkel et al. (2005) beschreiben ferner die Verabreichung von INCB3344 bei Ratten im Modell der Adjuvans-induzierten Arthritis. Die Verabreichung begann wiederum bereits zu einem Zeitpunkt (9 Tage nach Immunisierung), zu dem die Ratten noch keine oder nur geringe klinische Zeichen der Erkrankung zeigten.

Zusammenfassend ist festzustellen, dass es derzeit keine befriedigende Therapie für multiple Sklerose und/oder rheumatoide Arthritis gibt.

Aufgabe der vorliegenden Erfindung ist es vor diesem Hintergrund, eine neue Behandlungsmöglichkeit für multiple Sklerose und/oder rheumatoide Arthritis bereitzustellen.

Diese Aufgabe wird gelöst durch ein Medikament enthaltend einen Antikörpers, der an den Chemokinrezeptor CCR2 spezifisch binden kann, zur Behandlung von multipler Sklerose und/oder rheumatoider Arthritis bei einem Subjekt bzw. die Verwendung eines solchen Antikörpers zur Herstellung eines Medikamentes zur Behandlung von multipler Sklerose und/oder rheumatoider Arthritis bei einem Subjekt.

Überraschenderweise wurde im Rahmen der vorliegenden Erfindung gefunden, dass die Verabreichung von gegen CCR2 gerichteten Antikörpern im Gegensatz zur bisherigen Literaturmeinung (siehe insbesondere Brühl et al. (2004, oben zitiert) eine klinische Verbesserung von multipler Sklerose und/oder rheumatoider Arthritis bewirken kann. Es wurde nämlich festgestellt, dass durch Verabreichung von gegen CCR2 gerichteten Antikörpern sehr wohl eine klinische Verbesserung der rheumatoiden Arthritis erreicht werden kann, vorzugsweise bei einer Dosierung, die geringer ist, als die von Brühl et al. (2004) gewählte Dosis. Gleichermaßen wurde festgestellt, dass eine klinische Verbesserung der multiplen Sklerose bewirkt werden kann.

Ferner wurde überraschenderweise festgestellt, dass eine Behandlung nicht nur während der Auslösung der Erkrankung in einem Tiermodell möglich ist, sondern auch dann, wenn bereits deutliche klinische Symptome der Erkrankung vorliegen. Dies ermöglicht unter anderem eine relativ späte therapeutische Intervention, wie sie in der klinischen Praxis beim Menschen benötigt wird.

Der Chemokinrezeptor CCR2 ist dem Fachmann bekannt (siehe z.B. Eintrag Nr. 601267 in der Datenbank Online Mendelian Inheritance in Man (siehe OMIM auf der Website der National Institutes of Health, USA: http://www.ncbi.nlm.nih.gov/)). Es handelt sich hierbei um einen Chemokinrezeptor vom CC-Typ, der beim Menschen in zwei verschiedenen Splice-Varianten vorliegt, CCR2A und CCR2B (Wong, L.-M., Myers, S.J., Tsou, C.-L., et al. (1997) Organization and Differential Expression of the Human Monosite Chemoattractant Protein 1 receptor gene: Evidence for the role of the Carboxyl-Terminal Tail in Receptor Trafficking. J. Biol. Chem., Bd. 272, S. 1038-1045). Das Molekül ist auch unter dem Namen *Human Monocyte Chemoattractant Protein-1 receptor gene* bekannt. Es handelt sich hierbei um einen 7-Transmembrandomänen-G-Protein-gekoppelten Rezeptor. Die entsprechenden Orthologe von CCR2 aus etlichen anderen Spezies sind ebenfalls bekannt und können vom Fachmann beispielsweise mit Hilfe von Sequenzrecherchen ausgehend von dem humanen CCR2 leicht ermittelt werden.

Der Begriff des Antikörpers ist dem Fachmann bekannt. Gemäß der vorliegenden Erfindung ist der Begriff des Antikörpers in breitem Umfang zu verstehen und umfasst u.a. polyklonale, monoklonale und rekombinant hergestellte Antikörper, sowie Fragmente davon, wie z.B. Fv-, Fab-, und F(ab)₂-Fragmente, die einzelsträngig (single-chain) sein können. Vorzugsweise ist der Antikörper vom Isotyp IgG. Vorzugsweise ist der Antikörper von einem Isotyp, der in der Lage ist, Effektormechanismen wie beispielsweise ADCC (Antikörper-abhängige zelluläre Toxizität) oder Komplementaktivierung zu induzieren. Dies wäre z.B. bei human IgG1 oder Maus IgG2a der Fall. Vorzugsweise ist der Antikörper so beschaffen, dass er durch Immunsystem des Subjektes möglichst nicht oder nur in geringem Umfang abgestoßen wird. Dies kann beispielsweise dadurch erreicht werden, dass die nicht für die Erkennung des Antigens (hier des CCR2-Rezeptors) benötigten Regionen (z.B. die Fc-Regionen) aus Antikörper-Sequenzen der Spezies des Subjektes gebildet werden. Zum Beispiel sind dem Fachmann so genannte humanisierte Antikörper bekannt, die besonders für den Einsatz beim Menschen geeignet sind. Unter den Begriff des Antikörpers im Sinne der vorliegenden Erfindung fallen auch anderweitig modifizierte Antikörper, z.B. bispezifische Antikörper (siehe z.B. Schneider et al. (2005), oben bereits zitiert), Diabodies, sowie sogenannte Binder oder Aptamere, die beispielsweise mit Hilfe eines Peptid-Rückgrates oder eines Nukleinsäurerückgrates, z.B. einer RNA, gebildet werden können. Vorzugsweise haben die Antikörper im Sinne der vorliegenden Erfindung ein Molekulargewicht von weniger als 600 kDa, weiter bevorzugt von weniger als 300 kDa, noch weiter bevorzugt von weniger als 200 kDa und am meisdten bevorzugt von ungefähr 150 kDa. Die Herstellung von geeigneten polyklonalen, monoklonalen und rekombinanten Antikörpern, einschließlich der Herstellung von Bindern und Aptameren ist dem Fachmann bekannt (siehe z.B. Jörg Knäblein (Hrsg.), Modern Biopharmaceuticals, Bd. 2, S. 635), siehe auch Ausführungsbeispiel. Beispielsweise kann die Immunisierung durch Injektion von Zellen erfolgen, die den CCR2-Rezeptor exprimieren. Dies hat den Vorteil, dass der Rezeptor in seiner nativen Faltung als Antigen verwendet wird. Weiterhin ist eine Immunisierung mit PeptidFragmenten des Antigens möglich. Vorzugsweise werden entsprechende Peptide aus den extrazellulären Domänen von CCR2, insbesondere aus den ersten beiden Schleifen (Loops) gewählt. Eine Identifikation von geeigneten Antikörpern (einschließlich Bindern und Aptameren) ist auch durch Screening von Bibliotheken mit entsprechenden Molekülen oder Zellen, die diese Moleküle exprimieren, möglich, z.B. mittels Phage Display. Nach der Identifikation können die Antikörper (einschließlich Bindern und Aptameren) mittels dem Fachmann bekannter Verfahren hergestellt werden.

Vorzugsweise sind die erfindungsgemäßen Antikörper mit Domänen gekoppelt, die die depletierende Wirkung der Antikörper verstärken. Solche Domänen sind dem Fachmann bekannt. Ein Beispiel dafür ist eine Fc-Domäne, die ADCC oder Komplementaktivierung induziert. Eine andere Möglichkeit ist die Kopplung des Antikörpers mit einem Toxin. Der Antikörper kann aber auch ein bispezifischer Antikörper sein, wie z.B. die bispezifische Variante von MC-21, die in Schneider et al. (2005) beschrieben ist und als zweites Antigen das Maus CD3-Epsilon erkennt (Schneider et al., 2005, bereits oben zitiert).

Der Antikörper gemäß der vorliegenden Erfindung kann an den Chemokinrezeptor CCR2 binden. Vorzugsweise bindet der Antikörper an Loop1 und Loop2 der extrazellulären CCR2-Domänen. Vorzugsweise bindet der Antikörper mit einer Dissoziationskonstante Kd von 10⁷ M oder größer, mehr bevorzugt von 10⁸ M oder größer, noch bevorzugter von 10⁹ M oder größer, am meisten bevorzugt von 10¹¹ M oder größer. Vorzugsweise erfolgt die Bindung spezifisch. Unter spezifischer Bindung wird im Sinne der vorliegenden Erfindung verstanden, dass der Antikörper an CCR2 unter physiologischen Bedingungen (also beispielsweise in physiologischer Salzlösung, in Zellkultur oder in vivo, vorzugsweise im Blut oder Gewebe des betreffenden Subjektes) mit mindestens 10-facher Affinität, vorzugsweise 20-facher Affinität, mehr bevorzugt 50-facher Affinität, am meisten bevorzugt 100-facher Affinität an CCR2 im Vergleich zu anderen Proteinen bindet, insbesondere im Vergleich zu ähnlichen Proteinen, (z.B. im Vergleich zu CCR5, CCR1, CCR3, oder anderen Chemokinrezeptoren und G-Protein-gekoppelten Rezeptoren mit 7 Transmembrandomänen, vorzugsweise im Vergleich zu CCR5). Eine Bindung an andere Proteine kann jedoch tolerabel sein, sofern sie mit der therapeutischen Wirkung des Antikörpers nicht interferiert. Eine Kreuzreaktivität mit orthologen CCR2-Rezeptoren aus anderen Spezies ist jedoch möglich und kann sogar vorteilhaft sein, um einen Einsatz des Antikörpers in mehreren Spezies zu ermöglichen. Eine solche Kreuzreaktivität ist nicht ungewöhnlich und dem Fachmann ist bekannt, wie er die Kreuzreaktivität bestimmen kann. Weitere Angaben dazu sind auch dem Ausführungsbeispiel zu entnehmen.

Vorzugsweise kann der erfindungsgemäß zu verwendende Antikörper den Chemokinrezeptor CCR2 inhibieren. Ob ein Antikörper den Chemokinrezeptor CCR2 inhibiert, lässt sich einfach feststellen. Beispielsweise reduziert ein solcher Antikörper die durch MCP-1 und MCP-3 induzierte Chemotaxis von Zellen, die mit Hilfe von CCR2 chemotaktisch reagieren, z.B. Monozyten. Beispiele entsprechender Zellen aus Maus bzw. Mensch sind Monomac6-Zellen bzw. humane Monozyten aus periphärem Blut (PBMC). Ein entsprechender Assay ist im Ausführungsbeispiel beschrieben.

Ferner lässt sich die Inhibition des Chemokinrezeptors CCR2 dadurch feststellen, dass eine Inhibition der Bindung von MCP-1 und des von MCP-1 induzierten Calciumeinstroms erfolgt. Ein entsprechender Assay ist im Ausführungsbeispiel beschrieben.

Beispiele für Antikörper, die geeignet sind, oder einen Ausgangspunkt für abgewanderte Antikörper darstellen können, sind die Antikörper aus der Gruppe bestehend aus DOC-1, DOC-2, DOC-3 und MC-21. Die Herstellung der Antikörper DOC-1, DOC-2, DOC-3 sowie deren Eigenschaften sind im Ausführungsbeispiel beschrieben.

Das erfindungsgemäße Medikament enthält den Antikörper. Zusätzlich kann das Medikament jegliche Arten von Hilfsstoffen erhalten, die der Fachmann als angemessen erachtet. Solche Hilfsstoffe können beispielsweise Trägersubstanzen sein, wie z.B. Stärke, Lactose, Fette, Stearinsäure, Alkohol, physiologische Kochsalzlösungen oder andere Zusätze. Insbesondere kommen Hilfsstoffe in Frage, die Antikörper stabilisieren und haltbar machen.

Die Verabreichung des Medikamentes kann mit jedem bekannten Verfahren ausgeführt werden, mit welchem der im Medikament enthaltene Antikörper an die Zielzellen, d.h. insbesondere Monozyten, in vitro oder in vivo eingebracht werden kann. Beispielsweise kann das Medikament durch Injektion verabreicht werden, z.B. intravenös (i.v.), subkutan (s.c.) oder intraperitoneal (i.p.) in Form von Lösungen oder Infusionen. Aber auch andere Verabreichungsformen wie z.B. in mikroverkapselter Form oder in Form von Implantaten sind denkbar. Vorzugsweise erfolgt die Verabreichung des Medikamentes so, dass der Antikörper in den Blutkreislauf oder in das entsprechende Zielgebiet eintreten kann. Denkbar ist auch eine Verabreichung direkt in das Zielgebiet, beispielsweise bei multipler Sklerose in das Zentralnervensystem, z.B. die Cerebrospinalflüssigkeit, oder bei rheumatoider Arthritis in die betroffenen Gelenke.

Die Erkrankungen multiple Sklerose und rheumatoide Arthritis sind dem Fachmann bekannt und wurden bereits in der Einleitung beschrieben.

Der Begriff der Behandlung ist dem Fachmann ebenfalls bekannt. Im Sinne der vorliegenden Erfindung handelt es sich bei der Behandlung um jegliche Art von Intervention, die eine klinische Verbesserung der multiplen Sklerose und/oder rheumatoiden Arthritis bei einem Subjekt bewirken. Eine klinische Verbesserung kann beispielsweise bei der multiplen Sklerose z.B. durch Messung der Abnahme der neurologischen Ausfälle, z.B. der Lähmung, ermittelt werden. Bei der rheumatoiden Arthritis kann eine klinische Verbesserung z.B. durch Abnahme der Symptome, z.B. der Schwellung, der Entzündung der der Schmerzen ermittelt werden. Vorzugsweise äußert sich die Verbesserung in einer Milderung der klinischen Symptome, die beispielsweise einer Verbesserung des EAE-Scores gemäß dem Ausführungsbeispiel um mindestens 0,25 Einheiten, vorzugsweise mindestens 0,5 Einheiten, mehr bevorzugt mindestens 0,75 Einheiten, am meisten bevorzugt mindestens 1,0 Einheiten entspricht. Vorzugsweise äußert sich die Verbesserung in einer Milderung der klinische Symptome, die beispielsweise einer Verbesserung des Arthritis-Scores gemäß dem Ausführungsbeispiel um mindestens 0,25 Einheiten, vorzugsweise mindestens 0,5 Einheiten, mehr bevorzugt mindestens 0,75 Einheiten, mehr bevorzugt mindestens 1,0 Einheiten, am meisten bevorzugt mindestens 1,25 Einheiten entspricht. Für die Einschätzung der klinischen Verbesserung beim Menschen sind ebenfalls viele Parameter bekannt, beispielsweise der EDSS-Score für die Multiple Sclerose (Expanded Disability Status Scale, siehe Kurtzke, JF (1983). Rating neurologic impairment in multiple sclerosis: an expanded disablility status scale (EDSS). Neurology, Bd. 33(11), S. 1444-1452)). So kann sich die klinische Verbesserung in einer Milderung der Symptome äußern, die beispielsweise einer Verbesserung gemäß dem EDSS-Score um mindestens 0,5, vorzugsweise um mindestens 1,0, mehr bevorzugt mindestens 1,5 Einheiten, mehr bevorzugt mindestens 2,0 Einheiten, am meisten bevorzugt mindestens 2,5 Einheiten entspricht. Für die Einschätzung der klinischen Verbesserung beim Menschen ist ferner der ACR (American College of Rheumatology)-Score für die rheumatoide Arthritis bekannt.

Vorzugsweise betrifft die Behandlung ein Subjekt, das an multipler Sklerose und/oder rheumatoider Arthritis leidet. Insbesondere betrifft die Behandlung die multiple Sklerose und/oder rheumatoide Arthritis während der Erkrankung, insbesondere die Behandlung bei Vorliegen klinischer Zeichen der Erkrankung. Klinische Zeichen der Erkrankung entsprechen beispielsweise einem EAE-Score (siehe Ausführungsbeispiel) von 1,0 oder größer, bevorzugter 1,5 oder größer, noch bevorzugter 2,0 oder größer, noch bevorzugter 2,5 oder größer, insbesondere 3,0 oder größer. Klinische Zeichen der Erkrankung entsprechen ferner beispielsweise einem Arthritis-Score (siehe Ausführungsbeispiel) von 1,0 oder größer, bevorzugter 2 oder größer, noch bevorzugter 3 oder größer, noch bevorzugter 4 pro Pfote. Für die Einschätzung der klinischen Zeichen beim Menschen sind ebenfalls viele Parameter bekannt, beispielsweise der EDSS-Score (Expanded Disability Status Scale, siehe Kurtzke, JF (1983). Rating neurologic impairment in multiple sclerosis: an expanded disablility status scale (EDSS). Neurology, Bd. 33(11), S. 1444-1452)) sowie der ACR (American College of Rheumatology)-Score für die rheumatoide Arthritis.

Ferner kann die Behandlung insbesondere eine Therapie-refraktäre multiple Sklerose und/oder rheumatoide Arthritis betreffen, d.h. eine Form der jeweiligen Erkrankung, bei der mit den bisher bekannten Mitteln eine klinische Verbesserung nicht erreicht werden konnte. Bei einer nennenswerten Anzahl von Subjekten kommt es zu einem Verlauf bei dem mit bisherigen Mitteln keine klinische Verbesserung mehr möglich ist. Ferner kann die Behandlung ein Subjekt mit der jeweiligen Erkrankung betreffen, bei dem mit bisherigen Behandlungsmethoden unerwünschte Nebenwirkungen in nicht akzeptablem Maße auftreten. Ferner kann die Behandlung die jeweilige Erkrankung im fortgeschrittenen Stadium betreffen.

Das Subjekt im Sinne der vorliegenden Erfindung ist ein Wirbeltier, vorzugsweise ein Säugetier. Das Säugetier kann beispielsweise ein Nagetier (z.B. Maus, Ratte oder Kaninchen), ein Schwein, ein Hund, eine Katze oder Primat sein. Vorzugsweise ist das Säugetier ein Primat, (beispielsweise ein Makake oder Weißbüschelaffe oder ein Mensch) sein. Besonders bevorzugt ist das Subjekt ein Mensch.

Zur Verabreichung des Medikaments wird üblicherweise eine wirksame Dosis ermittelt. Der Begriff der wirksamen Dosis und die Ermittlung der wirksamen Dosis ist dem Fachmann bekannt, ferner kann er zur Ermittlung der wirksamen Dosis auf die hier zur Verfügung gestellten Angaben zurückgreifen. Als wirksam wird eine Dosis verstanden, bei der eine klinische Verbesserung der behandelten Erkrankung eintritt. Insbesondere wird im Sinne der vorliegenden Erfindung unter einer wirksamen Dosis eine Dosis des Medikamentes verstanden, die eine Milderung der Symptome der multiplen Sklerose und/oder der rheumatoiden Arthritis bei einem Subjekt bewirkt. Eine wirksame Dosis ist beispielsweise eine Dosis, die so gewählt wird, dass mindestens 20 %, vorzugsweise mindestens 30 %, mehr bevorzugt mindestens 40 %, am meisten bevorzugt mindestens 50 % der CCR2 exprimierenden Monozyten im peripheren Blut depletiert werden. Unter Depletion der Monozyten wird hierbei eine Zerstörung der Zellen verstanden.

Vorzugsweise wird die wirksame Dosis des Medikamentes so gewählt, dass sie die niedrigste Dosis darstellt, die einen befriedigenden Behandlungserfolg der multiplen Sklerose und/oder rheumatoiden Arthritis gewährleistet. Eine besonders geeignete wirksame Dosis kann dadurch ermittelt werden, dass in Testreihen die Dosis so lange erhöht wird, bis das gewünschte Verhältnis von Wirkung und unerwünschter Nebenwirkung erreicht ist. Im Rahmen der vorliegenden Erfindung wird dies beispielsweise erreicht sein, wenn die behandelte Erkrankung bei weiterer Erhöhung der Dosis nicht mehr weiter klinisch verbessert wird (ggf. sogar verstärkt wird) und/oder wenn unerwünschte Nebenwirkungen im Verhältnis zum therapeutischen Effekt nicht mehr akzeptabel sind.

Beispielsweise wäre eine Dosis zu erwägen, die nicht diejenige Dosis übersteigt, die notwendig ist, um eine Depletion von 99 %, vorzugsweise 98 %, bevorzugter 95 %, bevorzugter 90%, bevorzugter 80%, bevorzugter 70%, noch bevorzugter 50% der CCR2 exprimierenden Monozyten im peripheren Blut zu erreichen. Im Rahmen der Erfindung konnte gezeigt werden, dass eine Dosis, die zwar eine weitgehende Depletion der Monozyten bewirkt, aber auch nicht wesentlich höher ist, eine bemerkenswerte klinische Verbesserung und nicht etwa eine Verstärkung der Erkrankung bewirkt. Insbesondere betrifft dies die rheumatoide Arthritis.

Alternativ kann die Dosis daran orientiert werden, ob sie eine Freisetzung von Interleukin 6 (IL-6) und/oder Interleukin-4 (IL-4), bewirkt. Insbesondere kann diese Orientierung der Dosis an einer Freisetzung von IL-6 erfolgen. So kann die Dosis vorzugsweise so niedrig gewählt werden, dass sie nicht eine übermäßige Freisetzung von IL-6 und/oder IL-4 bewirkt. Beispielsweise kann die Dosis so gewählt werden, dass sie nicht mehr als eine 20fache Erhöhung, vorzugsweise nicht mehr als eine 10fache Erhöhung, mehr bevorzugt nicht mehr als eine 5fache Erhöhung, am meisten bevorzugt nicht mehr als eine 2fache Erhöhung des Spiegels von IL-6 und/oder IL-4 im Blutplasma bewirkt. In diesem Zusammenhang bezieht sich die Erhöhung auf den normalen Spiegel von IL-6 und/oder IL-4 im Blutplasma des Subjektes. Als "normaler" Spiegel von IL-6 und/oder IL-4 wird in diesem Zusammenhang vorzugsweise der Spiegel angesehen, den das Subjekt aufweist, bevor die Verabreichung des Medikamentes erfolgte. Es ist dem Fachmann bekannt, wann er eine Freisetzung von IL-6 und/oder IL-4 als durch die Gabe des Medikamentes bewirkt ansehen kann. Beispielsweise dürfte eine Erhöhung des Spiegels von IL-6 und/oder IL-4 zwei bis sechs Stunden nach intraperitonealer Injektion des Antikörpers als durch das Medikament bewirkt angesehen werden können.

Als geeignete Dosis ist ferner eine Dosis zu erwägen, deren untere Grenze wahlweise 0,01; 0,02; 0,03; 0,05; 0,07; 1,0; 1,5; 2,0; 2,5; oder 3,0 mg des Antikörpers pro kg Körpergewicht des Subjektes pro Tag entspricht und deren obere Grenze wahlweise 1,0; 1,5; 2,0; 2,5; 3,0; 4,0; 5,0; 7,0; 10; 12; 14; 17; oder 20 mg des Antikörpers pro kg Körpergewicht des Subjektes pro Tag entspricht, wobei die genannten Grenzen beliebig miteinander kombiniert werden können, dabei aber so ausgewählt werden, dass ein positiver Bereich von den Grenzen eingeschlossen wird. Beispiele für solche Bereiche sind 0,01-20 oder 0,01-2 oder 2,5-20 mg des Antikörpers pro kg Körpergewicht des Subjektes pro Tag.

Ferner betrifft die vorliegende Erfindung ein Medikament enthaltend einen Antikörper, der an den Chemokinrezeptor CCR2 binden kann, bei multipler Sklerose und/oder rheumatoider Arthritis bei einem Subjekt bzw. die Verwendung eines solchen Antikörpers zur Herstellung eines Medikamentes zum Depletieren von Monozyten bei multipler Sklerose und/oder rheumatoider Arthritis bei einem Subjekt.

Ferner betrifft die vorliegende Erfindung ein Medikament enthaltend einen Antikörper, der Monozyten depletieren kann, von multipler Sklerose und/oder rheumatoider Arthritis bei einem Subjekt bzw. die Verwendung eines solchen Antikörpers zur Herstellung eines Medikamentes zur Behandlung von multipler Sklerose und/oder rheumatoider Arthritis bei einem Subjekt.

Sämtliche der in dieser Beschreibung dargestellten bevorzugten Ausführungen und Varianten der Erfindung und Definitionen beziehen sich selbstverständlich auch auf die beiden vorgenannten Medikamente und Verwendungen in entsprechender Weise.

Im Rahmen der vorliegenden Erfindung wurde gefunden, dass die Verabreichung eines Antikörpers, der den Chemokinrezeptor CCR2 inhibieren kann, eine Depletion von CCR2 exprimierenden Monozyten bewirken kann.

Es wird von den Erfindern davon ausgegangen, dass die Behandlung der multiplen Sklerose und/oder rheumatoiden Arthritis vorzugsweise über eine Depletion von CCR2 exprimierenden inflammatorischen Monozyten erfolgt.

Unter dem Depletieren von Monozyten wird im Sinne der vorliegenden Erfindung die Zerstörung der entsprechenden Monozyten verstanden.

Der Mechanismus der Depletion kann durch die Gestaltung des Antikörpers beeinflusst werden. Vorzugsweise erfolgt die Depletion durch einen auf ADCC (Antikörper-abhängige zelluläre Toxizität) und/oder Komplementaktivierung basierenden Mechanismus der Zelllyse. Bei der Komplementaktivierung sollte allerdings darauf geachtet werden, dass es nicht zu einer verstärkten Entzündungsreaktion z.B. über die Komplementmoleküle C3a und C5a kommt. Die Depletion basierend auf ADCC kann beispielsweise über CD16/32 oder CD64 vermittelt sein. ADCC ermöglicht, dass Monozyten sich gegenseitig depletieren.

Eine Depletion mit Hilfe der erfindungsgemäßen Antikörper kann in den verschiedensten Spezies erreicht werden. So sind beispielsweise sind die Monozyten-Populationen von Maus und Mensch ähnlich. Beispielsweise ist bekannt, dass auch in der Maus eine Dichotomie der Blutmonozyten in inflammatorische (CD11b⁺ CCR2⁺ GR1⁺ CD62L⁺ CX₃CR₁^{low}) und nicht-inflammatorische (CD11b⁺ CCR2⁻ GR1⁻ CD62L⁻ CX₃CR₁^{high}) Monozyten beobachtet werden kann (Geissmann F, Jung S, Littman DR. (2003). Blood monocytes consist of two principal subsets with distinct migratory properties. Immunity, Bd. 19(1), S. 71-82.). Beim Menschen sind diese beiden Monozytenpopulationen schon länger bekannt und werden hauptsächlich über die Expressionshöhe der Oberflächenmarker CD14 und CD16 bestimmt (Ziegler-Heitbrock HW (2000). Definition of human blood monocytes. J Leukoc Biol., Bd. 67(5), S 603-6.).

In einer weiteren Ausführungsform kann für die Behandlung neben dem Antikörper gegen CCR2 zusätzlich auch noch mindestens ein Antikörper verwendet werden, der spezifisch für Monozyten ist, vorzugsweise ausgewählt aus der Gruppe bestehend aus CD14, CD33, CD64, CD68, CD91, CD115, und CD163. Besonders bevorzugt ist CD14. Eine solche Ausführungsform ist kann insbesondere vorteilhaft sein, wenn das Subjekt ein Primat ist, insbesondere ein Mensch.

In einer anderen Ausführungsform betrifft die vorliegende Erfindung ein Verfahren zum Depletieren von Monozyten umfassend das Kontaktieren der Monozyten mit einem Antikörper, der an den Chemokinrezeptor CCR2 spezifisch binden kann. Dabei können Verfahren ausgenommen sein, die eine chirurgische oder therapeutische Behandlung des menschlichen oder tierischen Körpers darstellen. Vorzugsweise erfolgt das Kontaktieren in vitro. Der Begriff "in vitro" wird in diesem Zusammenhang in seiner breitestmöglichen Form verstanden. Dabei bezieht er sich auf jegliches Verfahren, das außerhalb des lebenden Körpers erfolgt, also insbesondere auch auf Verfahren in Zellkultur, Gewebekultur oder Organkultur. Insbesondere wird unter dem Begriff in vitro auch ein Verfahren verstanden, das sich auf die Behandlung von Blut außerhalb des Körpers des Subjektes bezieht.

Die vorliegende Erfindung bezieht sich ferner auf ein Verfahren zur Behandlung von multipler Sklerose und/oder rheumatoider Arthritis bei einem Subjekt, umfassend den Schritt des Verabreichens eines Antikörpers, der an den Chemokinrezeptor CCR2 spezifisch binden kann.

Die vorliegende Erfindung bezieht sich ferner auf ein Verfahren zur Depletion von spezifischen Monozyten bei einem Subjekt, umfassend den Schritt des Verabreichens eines Antikörpers, der an den Chemokinrezeptor CCR2 spezifisch binden kann.

Sämtliche der oben bereits dargestellten bevorzugten Ausführungen und Varianten der Erfindung und Definitionen beziehen sich selbstverständlich auch auf die beiden vorgenannten Verfahren in entsprechender Weise.

### Beschreibung der Figuren:

### Fig.1

Kinetik der Monozytendepletion. Eine tägliche Injektion von 10 µg MC-21 bzw. Isotypkontrollantikörper führte zur Depletion von GR1+ Monozyten aus dem peripheren Blut von Mäusen für mindestens 5 Tage. Untersucht wurden 5 Mäuse pro Gruppe. Die Blutabnahmen erfolgten ca. 7 Stunden nach den Antikörperinjektionen. Am Verhältnis von GR1+ Monozyten zu CD19+ B-Zellen (die durch die Ak-Behandlung nicht wesentlich beeinflusst werden) erkennt man die für mind. 5 Tage anhaltende Depletion der GR1+ Monozyten durch den MC-21 Antikörper. day, Tag

### Fig.2

MC-21 induzierte in hoher Konzentration eine Freisetzung von IL-6. C57BL/6 Mäuse wurden mit 100 µg oder 10 µg MC-21 Antikörper intraperitoneal behandelt. 2 Stunden nach der Injektion wurden im Plasma die IL-6 Spiegel mittels ELISA (enzyme-linked immuno-sorbent assay) gemessen. Nur in hoher Dosis führte der MC-21 Antikörper zur Freisetzung von IL-6. anti-CCR2, MC-21 Antikörper; i.p., intra-peritoneal

### Fig. 3

A. Unterschiedliche MC-21 Konzentrationen modulierten differenziell den Verlauf der Arthritis. Im Modell der Kollagen-induzierten Arthritis (Immunisierung von DBA/1 Mäusen mit je 100 µg Kollagen am Tag 1 in kompletten Freundschen Adjuvans und an Tag 21 mit inkompletten Freundschen Adjuvans) führte die tägliche Gabe (Tag 21-32) von hochdosiertem MC-21 Antikörper (50 µg) zu einer deutlichen Verschlechterung der Arthritis, während die tägliche Gabe von niedrig dosiertem MC-21 (10 µg) zu einer deutlichen Verbesserung der Arthritis führte.
B. Die tägliche niedrig dosierte Gabe des MC-21 Antikörpers (anti-CCR2) (10-20 µg / Tag i.p.) war im Modell der Kollagen-induzierten Arthritis auch dann therapeutisch wirksam, wenn die Arthritis bereits deutlich fortgeschritten war. Der Antikörper MC-67 wurde als Isotyp-Kontrolle in gleicher Dosierung verwendet. Die Mäuse wurden 2-mal im Abstand von 3 Wochen mit Kollagen (200 µg in kompletten bzw. inkompletten Freundschen Adjuvans) immunisiert. Erst nach Auftreten von deutlichen Arthritis-Zeichen (Score 3) wurde mit der Antikörperbehandlung begonnen (Tag 0). Die Gabe von MC-21 führte zum Stillstand der Erkrankung, während in der Kontrollgruppe (Gabe von MC-67) die Erkrankung deutlich fortschreitet. Der Unterschied zwischen der MC-21- und MC-67-Gruppe war signifikant (p<0,05 für die Tage 1-6). Die Zunahme der Arthritis ab Tag 0 gerechnet war hochsignifikant in der MC-67 Gruppe. In der MC-Gruppe war die Veränderung der Arthritis nicht signifikant.

### Fig. 4

Die Depletion inflammatorischer Monozyten verbesserte den Verlauf der experimentellen autoimmunen Enzephalomyelitis (EAE) als Tiermodell der Multiplen Sklerose. Die Applikation von 20 µg MC-21, aber nicht von dessen Isotypkontrolle (IgG2b), verbesserte signifikant den Verlauf der Erkrankung. Der MC-21 Antikörper oder die Isotypkontrolle wurden ab dem Tag 17 (Höhepunkt der Erkrankung) täglich bis zum Tag 34 nach Immunisierung gegeben. Ein klinischer Schweregrad (score) von 2.5 entspricht einer deutlichen Hinterbeinschwäche mit einer unilateralen Hinterbeinlähmung. Ein Score von 1.0 entspricht einer Schwanzlähmung bei erhaltener Kraft/Motorik der Hinterbeine. daily, täglich; clinicial EAE score, klinischer EAE-Score; days after immunization, Tage nach Immunisierung

### Fig. 5

Geringerer Myelinschaden während der EAE in mit MC-21 behandelten Mäusen 35 Tage nach Immunisierung. Quantifizierung der Demyelinisierung. MC-21 behandelte Tiere hatten deutlich mehr erhaltenes Myelin. IgG2b, Isotypkontrolle; demyelination, Demyelinisierung

### Fig. 6

Die Injektion der Antikörper DOC-2 und DOC-3 führte im Gegensatz zur Injektion eines Isotypkontroll-Antikörpers (je 5 mg/kg Körpergewicht) zu einer Reduktion der Zahl der CCR2 positiven Monozyten im peripheren Blut von Weißbüschelaffen. Dargestellt ist der relative Anteil der CCR2 positiven Monozyten an der Gesamtzahl der Leukozyten, wobei dieser Wert in der mit Isotyp-Kontroll Antikörpern behandelten Gruppe für die jeweiligen Tage auf 100 % normiert wurde. CCR2⁺ peripheral blood monocytes, CCR2 positive Monozyten im peripheren Blut; injected antibody at 0 h, injizierter Antikörper bei 0 h; Isotyp-Ko, Isotypkontrolle

### Fig. 7

Die Depletion von inflammatorischen Monozyten in nicht-menschlichen Primaten führt zu einer Stabilisierung des Gewichts (A.) sowie zu einer Reduktion der neurologischen Defizite (B.). Adulte weibliche Weißbüschelaffen (Marmoset, *Callithrix jacchus*) wurden mit dem anti-humanen CCR2-Antikörper DOC-2 bzw. mit dem Isotyp Antikörper IgG1 (5mg/kg i.p.) behandelt. Mit der Applikation der Antikörper wurde begonnen, sobald sich bei den Tieren erste eindeutige neurologische Symptome manifestierten. Die Weißbüschelaffen wurden nachfolgend alle zwei Tage behandelt.

### Fig. 8

Die therapeutische Gabe des anti-humanen CCR2-Antikörpers führt zu einer signifikant verlängerten Lebenszeit, wenn mit der Behandlung am Krankheitshöhepunkt begonnen wird. Adulte weibliche Weißbüschelaffen (Marmoset, *Callithrix jacchus*) wurden mit dem anti-humanen CCR2-Antikörper DOC-2 (Anti-CCR2, 5 mg/kg i.p., n=2) bzw. PBS (n=2) ab dem Krankheitshöhepunkt und nachfolgend alle zwei Tage bis zum Tod behandelt.

Im folgenden Ausführungsbeispiel wird die Erfindung näher erläutert, ohne dass dies als eine Beschränkung des Umfangs der Erfindung verstanden werden soll.

### Ausführugsbeispiel:

Der MC-21 Antikörper als Mittel zur Depletion inflammatorischer Monozyten in vivo: Der monoklonale Antikörper MC-21 (Mack M, Cihak J, Simonis C, et al.. Expression and characterization of the chemokine receptors CCR2 and CCR5 in mice. J Immunol. 2001 vol. 166(7), pp. 4697-704) bindet spezifisch an murines CCR2 und zeigt keine Kreuzreaktion mit dem eng verwandten murinen CCR5 Rezeptor. Er zeigte eine starke Bindung an Monozyten, wobei hier die Subpopulation der GR1+ Monozyten in der Maus von dem Antikörper erkannt wird.

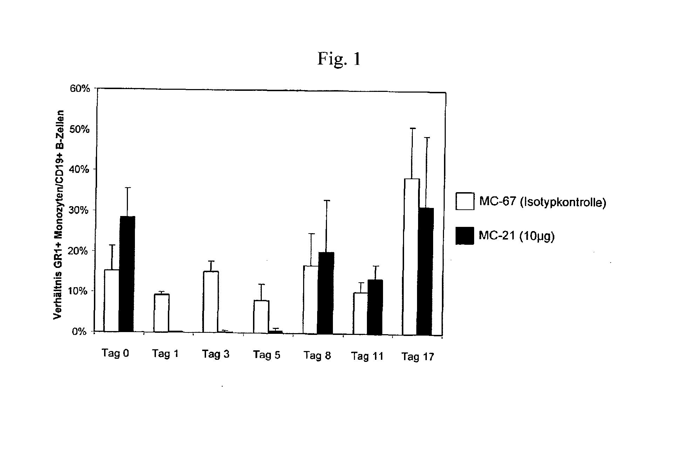

Wirkung und Kinetik des MC-21 Antikörpers in vivo: Eine einmalige intraperitoneale Injektion des MC-21 Antikörpers in C57BL/6 Mäuse führte zu einer praktisch vollständigen Depletion der inflammatorischen Subpopulation der Monozyten (CCR2+ GR1+ Monozyten) aus Blut und Milz (Fig. 1). In Abhängigkeit von der injizierten Dosis an MC-21 hielt die Depletion der Monozyten unterschiedlich lange an. Bei einer Injektion von 5-10 µg beobachteten wir nach 24 Stunden noch eine vollständige Depletion der GR1+ Monozyten, während nach 48 Stunden schon wieder ein Auftreten von GR1+ Monozyten zu verzeichnen war. Bei Injektion einer höheren Dosis an MC-21 (100 oder 500 µg) kam es zu einer für 2-3 Tage anhaltenden Monozytendepletion (entsprechend der längeren Präsenz des Antikörpers im Plasma). Durch wiederholte Injektionen von MC-21 Antikörper konnte eine Depletion von Monozyten für ca. eine Woche aufrechterhalten werden (Fig. 1). Danach kam es durch das Auftreten von inhibierenden Maus-anti-Ratte Antikörpern zu einem Verlust der CCR2 Bindungsfähigkeit der MC-21 Antikörper. Die MC-21 vermittelte Depletion von GR1+ Monozyten war weitgehend Fc-Rezeptor vermittelt, da eine gleichzeitige Injektion von Antikörpern gegen die Fc-Rezeptoren FcgRIII und FcgRII mittels anti-CD16/32 (Klon 2.4G2 ATCC HB197) die MC-21 vermittelte Depletion in Blut und Milz reduzierte. Es wird davon ausgegangen, dass der nicht durch CD16/32 Antikörper inhibierbare Anteil der MC-21 vermittelten Monozytendepletion durch einen Komplement-abhängigen Mechanismus oder durch einen auf CD64 beruhenden ADCC Mechanismus basiert.

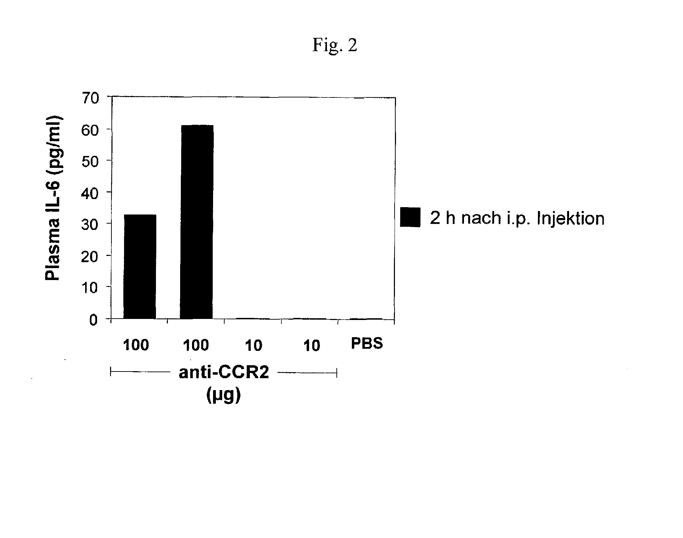

Der MC-21 Antikörper hatte auch Auswirkungen auf CCR2 exprimierende basophile Granulozyten. Inkubation von komplettem murinen Knochenmark mit dem MC-21 Antikörper führte zu einer deutlichen Freisetzung von IL-6. Diese IL-6 Freisetzung war abhängig von basophilen Granulozyten, da die Depletion der basophilen Granulozyten aus dem kompletten Knochenmark zu einer deutlich reduzierten MC-21 induzierten IL-6 Freisetzung führte. Auch isolierte basophile Granulozyten (Isolation mittels magnetischer Beads gegen DX-5 und anschließende FACS-Sortierung mittels CD45 und DX-5 Expression) wurden durch Inkubation mit MC-21 Antikörpern zur Freisetzung von IL-6 angeregt. Basophile Granulozyten von CCR2 defizienten Mäusen wurden durch MC-21 nicht zur IL-6 Freisetzung angeregt. Diese Daten zeigen, dass MC-21 eine Aktivierung von basophilen Granulozyten induzierte und dass diese Aktivierung durch Bindung von MC-21 an CCR2 stattfand. Da basophile Granulozyten auch eine starke Expression von Immunglobulin Fc-Rezeptoren zeigten (insbesondere CD16/32), gehen wir davon aus, dass diese Rezeptoren für die Immobilisierung von MC-21 wichtig sind und möglicherweise auch mit zur Basophilenaktivierung beitragen. In vivo war die Aktivierung von basophilen Granulozyten abhängig von der verabreichten Dosis an MC-21. Bei Injektion von 10 µg MC-21 beobachteten wir in vivo keine IL-6 Freisetzung, während bei Injektion von 100-500 µg eine deutliche IL-6 Freisetzung induziert wurde (Fig. 2).

Da eine Monozytendepletion bereits mit niedrigen MC-21 Dosen (z.B. 5-10 µg) zu erreichen war und IL-6 auf die Entwicklung einer Arthritis ungünstige Auswirkungen hatte, ergab sich durch wiederholte Injektion von MC-21 in niedriger Dosierung die Möglichkeit, eine Monozytendepletion ohne eine gleichzeitige Basophilenaktivierung durch MC-21 zu bewirken.

Für die Untersuchungen wurde das Modell der Kollagen-induzierten Arthritis (CIA) in männlichen DBA/1 Mäusen verwendet, einem Modell, das der humanen rheumatoiden Arthritis (RA) in vielen Punkten sehr nahe kommt und zur präklinischen Entwicklung wichtiger gegenwärtig in der Klinik verwendeter Therapien eingesetzt wird. In dem Modell der Kollagen-induzierten Arthritis wird die Arthritis induziert durch einmalige (Tag 1) oder zweimalige (Tag 1 und Tag 21) Immunisierung mit meist bovinem Kollagen.

Die Behandlung mit gegen CCR2 gerichteten Antikörpern ab Tag 1 des Erkrankungsmodells interferierte mit der Immunisierung mit Kollagen und deshalb fiel die Gelenkentzündung geringer aus.

Im Gegensatz zur inhibierenden Wirkung von anti-CCR2 Antikörpern zum Zeitpunkt der primären Immunisierung mit Kollagen bewirkte die Applikation des blockierenden CCR2 Antikörpers MC-21 bei erhöhter Konzentration (500 µg/Maus jeden 3. Tag) zu einem späteren Zeitpunkt (ab Tag 4, Tag 9, oder Tag 21) eine signifikante Verschlechterung des klinischen und histologischen Schweregrades. Dieser Effekt ging mit einer verstärkten humoralen Immunantwort gegen Kollagen (signifikant höhere anti-Kollagen Antikörpertiter) einher. Auch CCR2 defiziente Mäuse, die in den CIA-suszeptiblen Stamm DBA/1 zurückgekreuzt wurden, zeigten eine verstärkte Arthritis (Quinones MP, Ahuja SK, Jimenez F, Schaefer J, Garavito E, Rao A, Chenaux G, Reddick RL, Kuziel WA, Ahuja SS (2004). Experimental arthritis in CC chemokine receptor 2-null mice closely mimics severe human rheumatoid arthritis. J Clin Invest., Bd. 113(6), S. 856-66.). Diese Daten legten nahe, dass die Inhibition von CCR2 nach Induktion der Arthritis oder das Fehlen von CCR2 während des gesamten Krankheitsverlaufes zu einer deutlichen Verstärkung der Arthritis führte. CCR2 war nach dem Stand der Literatur deshalb als ungeeignetes Ziel für eine Behandlung von entzündlich rheumatischen Erkrankungen einzuschätzen.

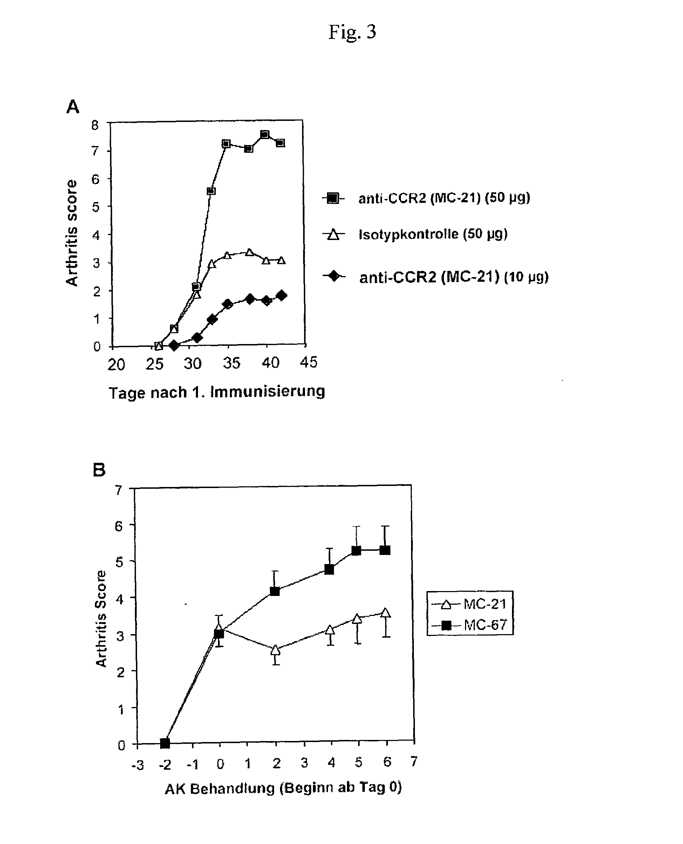

Überraschenderweise konnte im Rahmen der Erfindung nun gezeigt werden (Fig. 3A), dass eine Behandlung mit niedriger dosierten MC-21 Antikörpern (hier 10 µg intraperitoneal pro Tag) auch während der Progressionsphase der Kollagen-induzierten Arthritis zu einer deutlichen Verbesserung der Arthritis führte. MC-21 Antikörper wurden dabei erst ab Tag 21 gegeben. Im Vergleich dazu führte die tägliche Behandlung ab Tag 21 mit einer höheren Dosis an MC-21 (50 µg/Tag) zu einer deutlichen Verschlechterung der Arthritis. Auch eine Gabe von Antikörpern gegen IgE (ab Tag 21), die zu einer Aktivierung von basophilen Granulozyten führte, bewirkte eine deutliche Verschlechterung der Arthritis.

Monozytendepletion im MS-Modell: In dem Tiermodell der MS, der experimentellen autoimmunen Enzephalomyelitis (EAE), konnte die Erkrankung durch verschiedene Myelinkomponenten (z.B. MOG₃₅₋₅₅) oder durch adoptiven Transfer von autoreaktiven T-Zellen gegen diese Komponenten induziert werden.

In einer anderen Arbeit wurde gezeigt, dass ein selektiver kleinmolekularer CCR2-Antagonist den Verlauf der EAE positiv beeinflusst, wenn er vor dem Auftreten stärkerer Erkrankungssymptome gegeben wird (Brodmerkel CM, Huber R, Covington M, Diamond S, Hall L, Collins R, Leffet L, Gallagher K, Feldman P, Collier P, Stow M, Gu X, Baribaud F, Shin N, Thomas B, Burn T, Hollis G, Yeleswaram S, Solomon K, Friedman S, Wang A, Xue CB, Newton RC, Scherle P, Vaddi K (2005). Discovery and pharmacological characterization of a novel rodent-active CCR2 antagonist, INCB3344. J Immunol., Bd. 175(8), S. 5370-8.). Der zugrunde liegende Mechanismus könnte bei Brodmerkel und Kollegen möglicherweise eine reduzierte Migration von Monozyten ins Gehirn sein. Ob allerdings eine Hemmung der Monozytenmigration ins Gehirn auch noch therapeutisch wirksam ist, wenn zu einem späteren Zeitpunkt der Erkrankung bereits eine große Anzahl an Monozyten im Gehirn vorhanden ist, darf angezweifelt werden. Somit scheinen die Ergebnisse von Brodmerkel auf einer Beeinflussung der künstlichen Auslösung der Erkrankung in dem Modellsystem zu beruhen. Überraschenderweise wurde im Rahmen der Erfindung nun gezeigt, dass ein auf CCR2 basierendes Behandlungsregime im EAE-Stadium der Progression oder Effektorphase, d.h. während der Erkrankung, als Therapeutikum wirksam ist.

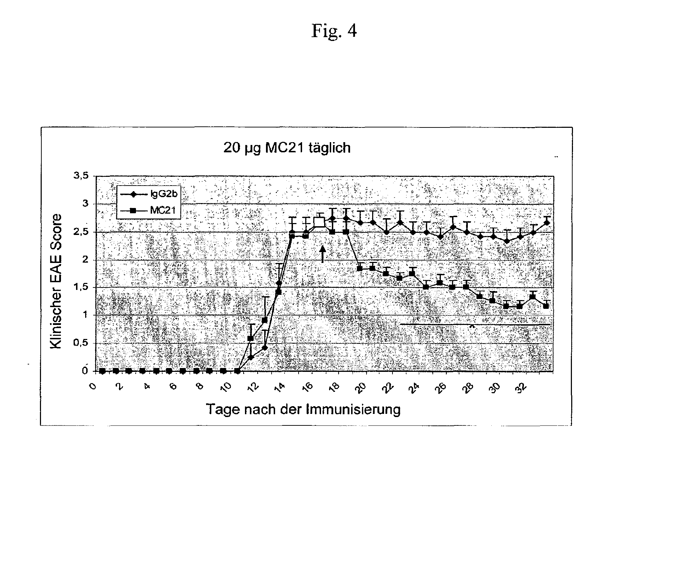

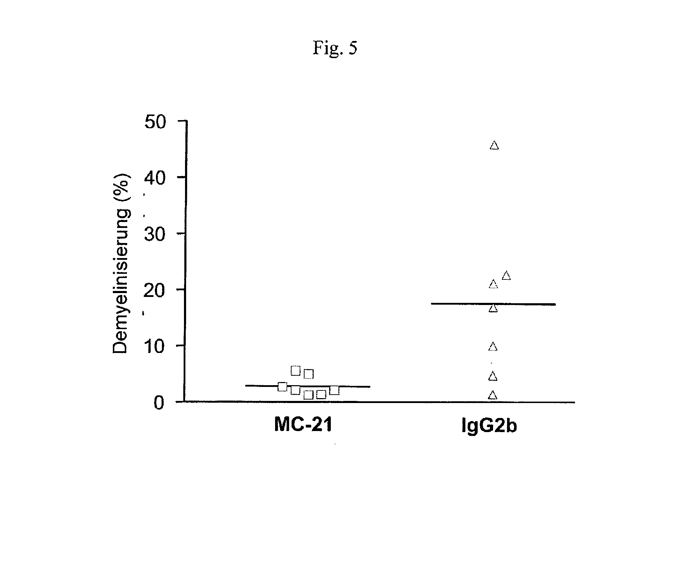

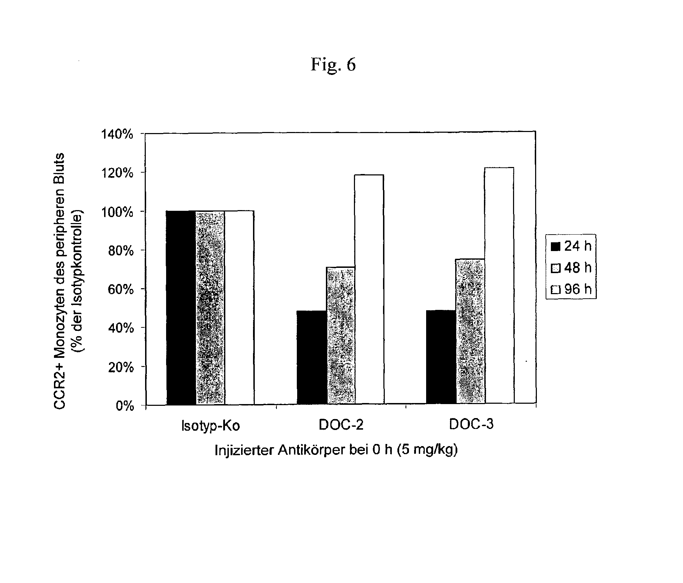

Es wurden 20 µg des monoklonalen anti-CCR2 Antikörpers MC-21 oder die entsprechende Isotypkontrolle (IgG2b) intraperitoneal täglich ab Tag 17 (Höhepunkt der Erkrankung) bis zum Tag 34 nach primärer Immunisierung, also beginnend vom Höhepunkt der Erkrankung (EAE Score bei 2,5, d.h. Hinterbeinschwäche mit unilateraler Beinlähmung). Es konnte eine deutliche und statistisch signifikante Reduktion des Krankheitsverlaufes beobachtet werden (Fig. 4). Ein klinischer Schweregrad 1.0 bedeutet eine Schwanzlähmung. Interessanterweise hatte im Gegensatz zum Arthritismodell auch eine vergleichsweise hohe Dosierung des MC-21 Ak (250 µg) eine Verbesserung des klinischen Verlaufes zur Folge. Eine funktionell relevante Basophilenaktivierung im EAE Modell durch MC-21 konnte weder durch eine Erhöhung von IL-6 im Serum behandelter Tiere noch durch eine Modulation des Krankheitsverlaufes durch anti-IgE-Gabe festgestellt werden.

Die sehr deutliche und statistisch signifikante Verbesserung des klinischen Schweregrades ging einher mit einer deutlichen Verringerung der infiltrierenden mononukleären Zellen im zentralen Nervensystem, wie am Beispiel der gesunkenen Anzahl CD3⁺ T Zellen und MAC-3⁺ Makrophagen in MC-21 behandelten Tieren am Tag 35 nach der Immunisierung gezeigt werden konnte.

Ebenso fiel der Gewebeschaden in behandelten Tieren deutlich geringer aus. So war z.B. der Grad der Demyelinisierung in MC-21 behandelten Tieren deutlich weniger ausgeprägt als in der Isotypkontrollgruppe.

Um festzustellen, welche Zytokine und Chemokine lokal im ZNS bei mit MC-21 behandelten und damit Monozyten-depletierten Tieren gebildet werden, wurde eine quantitative PCR durchgeführt. Das Rückenmark behandelter Tiere wurde am Tag 35 nach der Immunisierung entnommen die RNA extrahiert, revers transkribiert und mittels einer quantitativen real time (RT) PCR quantifiziert. Es fand sich eine deutlich geringere Produktion chemoattraktiver und toxischer Proteine, wie z.B. IP-10, MCP-1 und MIP-1a im ZNS von MC-21 behandelten Tieren.

In einem nächsten Schritt wurde untersucht, ob Primaten (Weißbüschelaffen, *Callithrix jacchus*) eine Kreuzreaktion mit Antikörpern gegen human CCR2 aufweisen. Als Antikörper gegen human CCR2 haben wir dabei die im Rahmen der Erfindung hergestellten Antikörper DOC-1, DOC-2 und DOC-3 analysiert.

Zur Herstellung der DOC-Antikörper wurden mindestens 6 Wochen alte BALB/c Mäuse 6-8 mal in mindestens 3-wöchigem Abstand mit mindestens 10 Mio. mit Full-length CCR2b stabil transfizierten CHO Zellen intraperitoneal immunisiert. Vier Tage nach der letzten Immunisierung wurden die Milzzellen mit X63.Ag8.653 Myelomzellen fusioniert und in Selektionsmedium kultiviert. Der Überstand der so entstandenen Hybridome wurde mittels FACS-Analyse auf Bindung an CCR2b-transfizierten bzw. als Kontrolle auf Bindung an CXCR4 transfizierten CHO Zellen getestet. Nach 2-maliger Reklonierung positiver Kulturen wurden mehrere monoklonale Antikörper (DOC-1, DOC-2 und DOC-3) erhalten, die spezifisch an human CCR2 binden und keine Kreuzreaktion mit murinem CCR2 oder CCR5, sowie mit humanen CCR1, CCR3, CCR5 oder CXCR4 aufweisen. Die DOC-Antikörper banden im humanen periphärem Blut auf einer Subpopulation der T-Zellen (ca. 20-30%), sowie auf der überwiegenden Zahl der CD14+ Monozyten (>95 %). Durch Präinkubation der humanen Zellen mit MCP-1 (1 µg/ml) für 30 min bei 37°C wurde die Bindung der CCR2 Antikörper auf den oben beschriebenen Leukozytensubpopulationen praktisch vollständig verhindert, was durch eine MCP-1 induzierte Internalisierung von CCR2 zu erklären ist. Dies zeigte zusätzlich die Spezifität der DOC-Antikörper.

Die DOC-Antikörper wurden hinsichtlich ihrer funktionellen Eigenschaften umfassend charakterisiert. Eine Zusammenfassung ist im folgendem dargestellt:

### Doc1:

besitzt den Isotyp IgG3
bindet an Loop1, 2 und 3 der extrazellulären CCR2-Domänen
induziert bei 37°C keine CCR2-Down-Modulation auf humanen Monozyten aus peripherem Blut (PBMC)
blockiert die MCP-1 induzierte CCR2-Down-Modulation auf humanen PBMC
blockiert die MCP-1 und MCP-3 induzierte Chemotaxis von Monomac6-Zellen und humanen PBMC
zeigt eine partielle Inhibition der MCP-1 Bindung und des MCP-1 induzierten Calciumeinstroms

### Doc2:

besitzt den Isotyp IgG1
bindet an Loop1 + erste Hälfte von Loop2 der extrazellulären CCR2-Domänen
induziert konzentrationsabhängig bei 37°C eine starke CCR2-Down-Modulation auf humanen PBMC
blockiert die MCP-1 und MCP-3 induzierte Chemotaxis von Monomac6-Zellen und humanen PBMC
zeigt eine sehr effektive Inhibition der MCP-1 Bindung und des MCP-1 induzierten Calciumeinstroms

### Doc3:

besitzt den Isotyp IgG1
bindet an Loop1 + zweite Hälfte von Loop2 der extrazellulären CCR2-Domänen
induziert konzentrationsabhängig eine schwache CCR2-Down-Modulation auf humanen PBMC
blockiert die MCP-1 und MCP-3 induzierte Chemotaxis von Monomac6-Zellen und humanen PBMC
zeigt eine sehr effektive Inhibition der MCP-1 Bindung und des MCP-1 induzierten Calciumeinstroms

Die Chemotaxis wurde in diesem Zusammenhang mit Hilfe von Transwell Filtereinsätzen (3 µm Porengröße für PBMC und 5 µm Porengröße für Monomac6) gemessen, wobei die Zellen (PBMC bzw. Monomac 6) in das obere well und die Chemokine (20-200 ng/ml) in das untere well gegeben wurden. Nach Inkubation für ca. 60 min. wurde die Zahl der in das untere well migrierten Zellen gemessen.

Der Calciumeinstrom wurde in diesem Zusammenhang mit Hilfe eines auf Äquorin und Lumineszenz basierenden Assay gemessen (siehe Blanpain et al, Molecular Biology of the Cell, Vol. 13, 723-737, 2002) Die Bindung der DOC-Antikörper an CCR2 wurde mittels Durchflusszytometrie gemessen, wobei als sekundäre Antikörper ein Farbstoff-markierter Kaninchen anti-Maus Ig Antikörper verwendet wurde.

Zur Bestimmung der Bindungsepitope der Antikörper wurde die Bindung an Zellen bestimmt, die chimäre Rezeptoren bestehend aus CCR2 und CCR5 exprimieren. Die Internalisierung (Down-Modulation) von CCR2 wurde ebenfalls mittels Durchflusszytometrie gemessen.

Die Kreuzreaktion der Antikörper DOC-1, DOC-2 und DOC-3 mit CCR2 auf Leukozyten von Weißbüschelaffen (*Callithrix jacchus*) wurde mittels FACS-Analyse untersucht. Dafür wurde Vollblut von Weißbüschelaffen mit 5 µg/ml der CCR2 Antikörper bzw. mit derselben Konzentration von Isotyp-Kontroll-Antikörpern (IgG1 und IgG3) inkubiert, gefolgt von einem PE (Phycoerythrin)-markierten sekundären Antikörper (Kaninchen (F(ab)2-Fragment anti-mouse Ig, R0439, DakoCytomation). Nach Lyse der Erythrozyten erfolgte die FACS-Analyse. Um die Spezifität der Bindung der DOC-Antikörper an Weißbüschelaffen-CCR2 zusätzlich zu belegen, wurde bei einem Teil des Vollblutes vor Inkubation mit den DOC-Antikörpern eine Internalisierung von CCR2 mit humanem MCP-1 induziert. Dafür wurde das Vollblut mit 1 µg/ml MCP-1 für 30 min bei 37°C präinkubiert und anschließend wie oben beschrieben auf Bindung der DOC-Antikörper untersucht. Es zeigte sich eine starke Bindung der Antikörper DOC-2 und DOC-3 und eine etwas geringere Binding des Antikörpers DOC-1 auf der im forward-sideward scatter des FACS dot plots abzugrenzenden Monozytenpopulation. Etwa 50 % der so abgegrenzten Monozyten banden DOC-2, DOC-3 und DOC-1. Diese Bindung ging vollständig verloren, wenn MCP-1 präinkubierte Leukozyten verwendet wurden. Die Isotypkontrollantikörper zeigten keine Bindung an Monozyten. Abgesehen von der Monozytensubpopulation zeigte sich eine Bindung der DOC-Antikörper lediglich auf einer sehr kleinen weiteren Leukozytensubpopulation, die aufgrund ihres forward-sideward scatters als basophile Granulozyten einzuordnen sind. Die von den DOC-Antikörpern erkannte Monozytenpopulation war homogen CD11b positiv, was durch Färbung mit dem Antikörperklon (M1/70, rat-anti mouse CD11b) gezeigt wurde. Dieser CD11b Antikörper zeigte eine Kreuzreaktion mit humanem und auch Weißbüschelaffen-CD11b.

Zusätzlich wurden die pharmakokinetische Eigenschaften der DOC Antikörper untersucht. 24, 48 und 96 Stunden nach Injektion der Antikörper DOC-2 und DOC-3 (5 mg/kg Körpergewicht) wurden die Plasmakonzentrationen der Antikörper gemessen. Dafür wurden Plasma der Affen in verschiedenen Verdünnungen mit hCCR2b transfizierten CHO-Zellen inkubiert und anhand einer Standardkurve, bestehend aus verschiedenen Verdünnungen der Antikörper DOC-2 und DOC-3, die Plasmakonzentration der DOC-Antikörper errechnet:

| Plasmakonzentration | 24 h | 48 h | 96 h |
|---|---|---|---|
| (µg/ml) | nach Injektion | nach Injektion | nach Injektion |
| DOC-2 | 159 | 85 | 3,3 |
| DOC-3 | 20 | 6,3 | 0,9 |

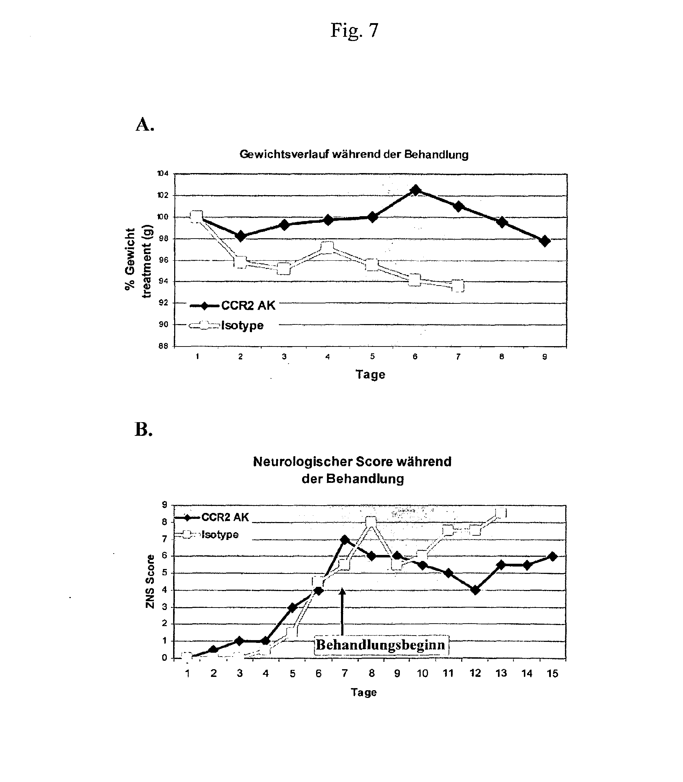

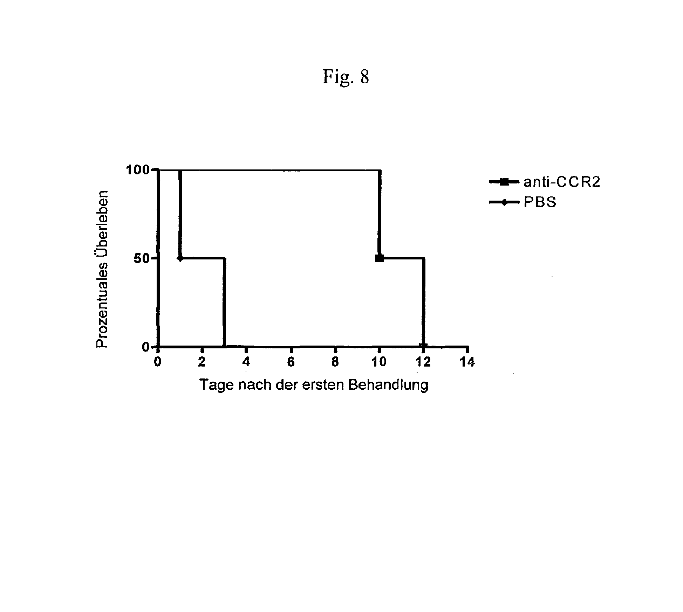

Außerdem wurde untersucht, ob es nach Injektion der DOC-2 bzw. DOC-3 Antikörper zu einer Depletion der CCR2 positiven Monozyten kam. Dafür wurde 24h, 48h und 96h nach Injektion der Antikörper DOC-2 und DOC-3 bzw. eines Isotyp-Kontroll Antikörpers (jeweils 5 mg/kg Körpergewicht, je 2 Affen pro Gruppe) Blut abgenommen und der Anteil der CCR2 positiven Monozyten an der Gesamtzahl der Leukozyten mittels FACS-Analyse bestimmt. Vollblut wurde dazu, wie oben beschrieben, mit den Antikörpern DOC-2 bzw. DOC-3 gefolgt von einem Phycoerythrin-markierten Sekundärantikörper gefärbt und in Verbindung mit dem forward-sideward scatter die CCR2 positiven Monozyten identifiziert. Fig. 7 zeigt, dass der Anteil der CCR2 positiven Monozyten an der Gesamtzahl der Leukozyten in den mit DOC-2 oder DOC-3 behandelten Tieren am ersten Tag nach Injektion reduziert war (auf ca. 50%). Der Anteil der CCR2 positiven Monozyten an der Gesamtzahl der Leukozyten in der mit Isotypkontroll-Antikörpern behandelten Gruppe wurde zu den jeweiligen Tagen gleich 100 % gesetzt.

Nachdem die Effizienz des anti-humanen DOC-2 AK mit einer hochgradigen Depletion von inflammatorischen Monozyten im peripheren Blut von Weißbüschelaffen gezeigt werden konnte, wollten wir die Wirksamkeit dieses depletierenden AK im EAE-Krankheitsmodell am Primaten untersuchen. Dazu wurden adulte (ca. 3 Jahre alte) weibliche Weißbüschelaffen (Marmoset, *Callithrix jacchus)* mit dem rekombinanten Ratten-MOG Protein in *Komplettem Freundschen Adjuvans* (KFA) immunisiert (50 µg MOG in 600 µl KFA). Nach 3-4 Wochen begannen die Tiere erste neurologische Symptome und Inappetenz zu zeigen. Wir begannen die therapeutische Applikation mit dem anti-humanen CCR2-Antikörper DOC-2 bzw. der entsprechenden Isotyp-Kontrolle (IgG1) bei den ersten eindeutigen neurologischen Krankheitssymptomen. Anschließend wurden die Weißbüschelaffen alle zwei Tage mit DOC-2 AK oder Isotyp AK (5 mg/kg i.p.) behandelt. Die mit dem DOC-2 AK behandelten Primaten zeigten während der Therapie im Gegensatz zu der Kontrolle eine Stabilisierung des Gewichts sowie einen Rückgang der neurologischen Defizite. In einem weiteren Experiment, bei dem wir mit der Applikation der Antikörper am Krankheitshöhepunkt starteten, konnten wir eine signifikant längere Überlebenszeit für die letal verlaufende EAE im Vergleich zu den Kontrolltieren nachweisen.

Zusammenfassend zeigen die im Rahmen der Erfindung ermittelten Daten erstmals, dass ein Antikörper, der CCR2+, GR1+ inflammatorische Monozyten in vivo depletierte, sowohl im Mausmodell und Primatenmodell der MS als auch im Mausmodell der Arthritis einen deutlichen therapeutischen Effekt besaß.

FACS-Analyse: Mit EDTA antikoaguliertes Vollblut bzw. Splenozyten wurden für 45 min auf Eis mit direkt markierten oder unmarkierten Antikörpern inkubiert und anschließend durch 3 maliges Waschen entfernt. Als direkt markierte Antikörper (Markierung mit den Farbostoffen FITC, PE, PE-Cy5, APC) gegen murine Zellen wurden verwendet: Antikörper gegen CD11b, GR1, CD4, CD19, CD45, DX5, IgE (alle von BD-Pahrmingen). Monozyten wurden anhand ihres forward- und sideward light scatters und anhand ihrer Expression von CD11b identifiziert und nach Expression von Gr1 in Gr1+ und GR1-Monozyten unterteilt.

Als nicht direkt markierte Antikörper wurden verwendet der CCR2-Antikörper MC-21 (5 µg/ml) bzw. eine Ratte-IgG2b Isoytpkontroll-Antikörper (5 µg/ml, BD-Pharmingen), gefolgt von einem biotinyliertem Maus-anti-Ratte-IgG2b (5 µg/ml, BD-Pharmingen), gefolgt von 10% Rattenserum, gefolgt von Streptavidin-PE bzw. Streptavidin-PE-Cy5 (DakoCytomation) und ggf. weiteren direkt markierten Antikörpern. Die unmarkierten Antikörper DOC-1, DOC-2 und DOC-3 bzw. die entsprechenden Isotypkontrollen (Sigma-Aldrich) wurden gefolgt von einem PE-markierten polyklonalen Kanninchen anti-Maus F(ab)2 Fragment (DakoCytomation, R0439).

Nach Färbung fand eine Erythrozytenlyse mit FACS-Lysing Solution (BD-Pharmingen) statt. Die FACS Analyse wurde durchgeführt auf einem FacsCalibur Gerät von BD-Pharmingen.

ELISA: Die Messung von IL-6 im Kulturüberstand bzw. EDTA-Plasma wurde mit einem kommerziellen ELISA (OptEIA)von BD-Pharmingen durchgeführt.

Zellisolation/Zelldepletion: Naive Basophile wurden isoliert durch Anreicherung von DX-5 positiven Zellen aus Milz und Knochenmark von Mäusen mittels DX-5 magnetischen Beads (Miltenyi, Isolation nach Anleitung). Anschließend fand eine Färbung mit DX-5-APC und CD45-FITC statt, anhand derer mittels FACS-Sort (BD, FACS ARIA) die basophilen Granulozyten (DX5+, CD45 low) isoliert wurden. Die Reinheit der so gewonnenen basophilen Granulozyten betrug über 95 %.

Zur Depletion von basophilen Granulozyten wurden die Ausgangszellen mit anti-IgE-FITC Antikörpern inkubiert, gewaschen und anschließend mit anti-FITC Beads inkubiert und über magnetische Sortierung depletiert (Miltenyi). Die Depletion betrug über 95 %.

Kollagen-induzierte Arthritis: Mindestens 8 Wochen alte männliche DBA/1 Mäuse wurden am Tag 1 mit 100 µg bovinem Kollagen (Sigma, C1188) in kompletten Freundschen Adjuvans und am Tag 21 mit 100 µg Kollagen in inkompletten Freundschen Adjuvans subkutan an der Schwanzbasis immunisiert. Der klinische Arthritis-Score wurde bestimmt, indem für jede Pfote ein Score erhoben wird, der für alle 4 Pfoten aufaddiert wird: Score pro Pfote: 1 bei Schwellung eines Gelenks, 2 bei Schwellung von mindestens 2 Gelenken, 3 bei Schwellung der gesamten Pfote, 4 bei Deformation der Pfote. Die Behandlung mit Antikörpern erfolgte wie in Abbildungen beschrieben.

Experimentelle autoimmune Enzephalomyelitis (EAE) in der C57BL/6 Maus: Für eine aktive Immunisierung wurden 6-8 Wochen alte Mäuse subkutan mit 200 µg des MOG₃₅₋₅₅ Peptids, emulgiert in CFA (complete Freund's Adjuvans) (angereichert mit 1mg/ml von Mycobacterium tuberculosis (Difco)), gespritzt; gefolgt von 250 ng Pertussistoxin i.v. am gleichen Tag und 2 Tage später. Die klinische Manifestation der EAE begann gewöhnlich zwischen dem 13. und 18. Tag nach Immunisierung. Die Mäuse wurden dann 4 x wöchentlich nach folgenden Kriterien neurologisch eingeschätzt: 0, keine sichtbaren neurologischen Symptome; 0.5, Schlaffheit des distalen Schwanzes; 1, Schlaffheit des kompletten Schwanzes; 1.5; Schlaffheit des kompletten Schwanzes und Schwäche der Hinterbeine; 2, unilaterale partielle Parese der Hinterbeine; 2.5, bilaterale partielle Parese der Hinterbeine; 3, komplette Parese der Hinterbeine; 3.5 komplette Parese der Hinterbeine und unilaterale Parese der Vorderbeine; 4, komplette Parese aller 4 Extremitäten; 5, tot.

Bei der EAE im Weißbüschelaffen *(Callithrix jacchus)* wurde rekombinantes Ratten-MOG in Komplettem Freundschen Adjuvans (KFA) emulgiert (50µg MOG in 600 µl KFA). In Portionen von jeweils 150 µl wurde die Emulsion am Rücken an vier Stellen subkutan appliziert. Zwei Applikationsstellen waren im Schulter- und die anderen beiden im Beckenbereich. Vor der Applikation wurden die entsprechenden Körperstellen rasiert und die Haut desinfiziert.

Statt M. tuberculosis verwendeten wir KFA Mycobacterium buytricum in einer reduzierten Dosis von 1 mg/ml KFA. Durch diese Modifikationen wurde das normalerweise stark wirksame KFA abgeschwächt ohne jedoch seine Wirkung zu verlieren. Diese Immunanstimulation war notwendig, um die Autoimmunreaktion auszulösen.

Real-Time PCR (RT-PCR): Zu diesem Zweck wurden die Tiere getötet, das Rückenmark frisch entnommen und mit sterilem PBS kurz gespült. Die totale RNA wurde mittels Trizol Reagenz entsprechend der Herstellerangaben isoliert in cDNA umgeschrieben und die RT-PCR durchgeführt.

Histologie: Die Mäuse wurden mittels CO₂ getötet. Die Wirbelsäule wurde entnommen und in 10% gepuffertes Formalin gegeben. Nach der Fixierung wurde das Rückenmark entnommen, eingebettet und entweder mit H&E zur Einschätzung der Leukozyteninfiltration oder mit Luxol fast blue (LFB) zur Darstellung der myelinisierten Fasern gefärbt. Andere immunohistochemische Färbungen schlossen verschiedene Blutzelltypen ein (MAC-3 für Makrophagen: BD Pharmingen; CD3 für T Zellen: Serotec, Düsseldorf).

Die folgenden Beschreibungsseiten 26 bis 29 enthalten bevorzugte Ausführungsbeispiele. Entsprechend bezieht sich der wie hierin verwendete Begriff "Anspruch" auf solch ein bevorzugtes Ausführungsbeispiel.
1. Medikament enthaltend einen Antikörper, der an den Chemokinrezeptor CCR2 spezifisch binden kann, zur Behandlung von multipler Sklerose und/oder rheumatoider Arthritis bei einem Subjekt.
2. Medikament gemäß Anspruch 1, wobei das Subjekt ein Primat, vorzugsweise ein Mensch, ist.
3. Medikament gemäß einem der Ansprüche 1 bis 2, zur Behandlung von Therapie-refraktärer multipler Sklerose und/oder von multipler Sklerose in einem fortgeschrittenen Stadium.
4. Medikament gemäß einem der Ansprüche 1 bis 2, zur Behandlung von Therapie-refraktärer rheumatoider Arthritis und/oder rheumatoider Arthritis in einem fortgeschrittenen Stadium.
5. Medikament gemäß einem der Ansprüche 1 bis 4, wobei der Antikörper gewählt ist aus der Gruppe bestehend aus Doc-1, Doc-2, Doc-3, und MC-21.
6. Medikament gemäß einem der Ansprüche 1 bis 5, wobei die Behandlung mit einer wirksamen Dosis des Medikamentes erfolgt, die so gewählt wird, dass sie nicht diejenige Dosis übersteigt, die notwendig ist, um eine Depletion von 95%, vorzugsweise 90%, bevorzugter 80% der CCR2 exprimierenden Monozyten im peripheren Blut zu erreichen.
7. Medikament gemäß Anspruch 6, wobei die Behandlung mit einer wirksamen Dosis des Medikamentes erfolgt, die so gewählt wird, dass mindestens 20%, vorzugsweise mindestens 30%, der CCR2 exprimierenden Monozyten im peripheren Blut depletiert werden.
8. Medikament gemäß einem der Ansprüche 1 bis 5, wobei die Behandlung mit einer wirksamen Dosis des Medikamentes erfolgt, die so gewählt wird, dass sie nicht mehr als eine 20fache Erhöhung, vorzugsweise nicht mehr als eine 1 0fache Erhöhung, des Spiegels von Interleukin-6 (IL-6) und/oder Interleukin-4 (IL-4) im Blutplasma bewirkt.
9. Medikament gemäß einem der Ansprüche 1 bis 5, wobei die Behandlung mit einer wirksamen Dosis des Medikamentes erfolgt, die so gewählt wird, dass sie 0,01-20 mg des Antikörpers pro kg Körpergewicht des Subjektes pro Tag entspricht.
10. Medikament enthaltend einen Antikörper, der Monozyten depletieren kann, zur Behandlung von multipler Sklerose bei einem Subjekt.
11. Medikament gemäß Anspruch 10, wobei die Medikament wie in einem oder mehreren der Ansprüche 2 bis 3 und 5 bis 9 weiter definiert ist.
12. Medikament enthaltend einen Antikörper, der Monozyten depletieren kann, zur Behandlung von rheumatoider Arthritis bei einem Subjekt.
13. Medikament gemäß Anspruch 12, wobei die Medikament wie in einem oder mehreren der Ansprüche 2 und 4 bis 9 weiter definiert ist.
14. Verfahren zum Depletieren von Monozyten, umfassend das Kontaktieren der Monozyten mit einem Antikörper, der an den Chemokinrezeptor CCR2 binden kann, insbesondere ausgenommen Verfahren, die eine chirurgische oder therapeutische Behandlung des menschlichen oder tierischen Körpers darstellen.
15. Verfahren zur Behandlung von multipler Sklerose und/oder rheumatoider Arthritis bei einem Subjekt, umfassend den Schritt des Verabreichens eines Antikörpers, der an den Chemokinrezeptor CCR2 spezifisch binden kann.
16. Verfahren gemäß Anspruch 15, wobei das Subjekt ein Primat, vorzugsweise ein Mensch, ist.
17. Verfahren gemäß einem der Ansprüche 15 bis 16, wobei es sich bei der multiplen Sklerose um eine Therapie-refraktäre multiple Sklerose und/oder um eine multiple Sklerose in einem fortgeschrittenen Stadium handelt.
18. Verfahren gemäß einem der Ansprüche 15 bis 16, wobei es sich bei der rheumatoiden Arthritis um eine Therapie-refraktäre rheumatoide Arthritis und/oder um eine rheumatoide Arthritis in einem fortgeschrittenen Stadium handelt.
19. Verfahren gemäß einem der Ansprüche 15 bis 18, wobei der Antikörper gewählt ist aus der Gruppe bestehend aus Dos-1, Doc-2, Doc-3, und MC-21.
20. Verfahren gemäß einem der Ansprüche 15 bis 19, wobei die Behandlung mit einer wirksamen Dosis erfolgt, die so gewählt wird, dass sie nicht diejenige Dosis übersteigt, die notwendig ist, um eine Depletion von 95%, vorzugsweise 90%, bevorzugter 80% der Monozyten im peripheren Blut zu erreichen.
21. Verfahren gemäß Anspruch 20, wobei die Behandlung mit einer wirksamen Dosis erfolgt, die so gewählt wird, dass mindestens 20%, vorzugsweise mindestens 30%, der Monozyten im peripheren Blut depletiert werden.
22. Verfahren gemäß einem der Ansprüche 15 bis 19, wobei die Behandlung mit einer wirksamen Dosis erfolgt, die so gewählt wird, dass sie nicht eine mehr als 20fache Erhöhung, vorzugsweise nicht mehr als eine 10fache Erhöhung, des Spiegels von Interleukin-6 (IL-6) und/oder Interleukin-4 (IL-4) im Blutplasma bewirkt.
23. Verfahren gemäß einem der Ansprüche 15 bis 19, wobei die Behandlung mit einer wirksamen Dosis erfolgt, die so gewählt wird, dass sie 0,01-20 mg des Antikörpers pro kg Körpergewicht des Subjektes pro Tag entspricht.
24. Verfahren zur Depletion von Monozyten bei einem Subjekt, umfassend den Schritt des Verabreichens eines Antikörpers, der an den Chemokinrezeptor CCR2 spezifisch binden kann.
25. Verfahren gemäß Anspruch 24, wobei mit dem Verfahren eine multiple Sklerose und/oder rheumatoide Arthritis bei dem Subjekt behandelt wird.
26. Verwendung eines Antikörpers, der an den Chemokinrezeptor CCR2 spezifisch binden kann, zur Herstellung eines Medikaments zur Behandlung von multipler Sklerose und/oder rheumatoider Arthritis bei einem Subjekt, wobei das Medikament vorzugsweise wie in einem beliebigen der Ansprüche 1 bis 9 definiert ist.
27. Verwendung eines Antikörpers, der Monozyten depletieren kann, zur Herstellung eines Medikaments zur Behandlung von multipler Sklerose und/oder rheumatoider Arthritis bei einem Subjekt, wobei das Medikament vorzugsweise wie in einem beliebigen der Ansprüche 10 bis 13 definiert ist.

## Patentansprüche

1. Medikament enthaltend einen Antikörper, der spezifisch an Loop 1 und Loop 2 der extrazellulären Domäne des Chemokinrezeptor CCR2 binden kann, zur Verwendung in der Behandlung von multipler Sklerose und/oder rheumatoider Arthritis bei einem Subjekt, wobei die Behandlung mit einer wirksamen Dosis des Medikamentes erfolgt.

2. Medikament zur Verwendung gemäß Anspruch 1, wobei das Subjekt ein Primat, vorzugsweise ein Mensch, ist.

3. Medikament zur Verwendung gemäß Anspruch 1 oder 2, wobei die Behandlung eine Behandlung von Therapie-refraktärer multipler Sklerose und/oder von multipler Sklerose in einem fortgeschrittenen Stadium oder eine Behandlung von Therapie-refraktärer rheumatoider Arthritis und/oder rheumatoider Arthritis in einem fortgeschrittenen Stadium ist.

4. Medikament zur Verwendung gemäß einem beliebigen der Ansprüche 1 bis 3, wobei der Antikörper ausgewählt ist aus der Gruppe bestehend aus Doc-2 und Doc-3.

5. Medikament zur Verwendung gemäß einem beliebigen der Ansprüche 1 bis 4, wobei die Behandlung mit einer wirksamen Dosis des Medikamentes erfolgt, die so gewählt wird, dass sie nicht diejenige Dosis übersteigt, die notwendig ist, um eine Depletion von 95%, vorzugsweise 90%, bevorzugter 80% der CCR2 exprimierenden Monozyten im peripheren Blut zu erreichen, insbesondere wobei die Behandlung mit einer wirksamen Dosis des Medikamentes erfolgt, die so gewählt wird, dass mindestens 20%, vorzugsweise mindestens 30%, der CCR2 exprimierenden Monozyten im peripheren Blut depletiert werden.

6. Medikament zur Verwendung gemäß einem beliebigen der Ansprüche 1 bis 4, wobei die Behandlung mit einer wirksamen Dosis des Medikamentes erfolgt, die so gewählt wird, dass sie nicht mehr als eine 20fache Erhöhung, vorzugsweise nicht mehr als eine 10fache Erhöhung, des Spiegels von Interleukin-6 (IL-6) und/oder Interleukin-4 (IL-4) im Blutplasma bewirkt.

7. Medikament zur Verwendung gemäß einem beliebigen der Ansprüche 1 bis 4, wobei die Behandlung mit einer wirksamen Dosis des Medikamentes erfolgt, die so gewählt wird, dass sie 0,01-20 mg des Antikörpers pro kg Körpergewicht des Subjektes pro Tag entspricht.

8. Medikament zur Verwendung gemäß einem beliebiegen der Ansprüche 1-7, wobei der Antikörper vom IgG Isotyp ist, vorzugsweise humanem IgG1 oder Maus IgG2a.

9. Medikament zur Verwendung gemäß einem beliebigen der Ansprüch 1-8, wobei das Subjekt ein Mensch und der Antikörper ein humanisierter Antikörper ist.

10. Verfahren zum Depletieren von Monozyten, umfassend das Kontaktieren der Monozyten mit einem Antikörper, der spezifisch an Loop 1 und Loop 2 der extrazellulären Domäne des Chemokinrezeptor CCR2 binden kann, ausgenommen Verfahren, die eine chirurgische oder therapeutische Behandlung des menschlichen oder tierischen Körpers darstellen.

11. Verwendung eines Antikörpers, der spezifisch an Loop 1 und Loop 2 der extrazellulären Domäne des Chemokinrezeptor CCR2 binden kann, in einem Verfahren zum Depletieren von Monozyten, ausgenommen Verfahren, die eine chirurgische oder therapeutische Behandlung des menschlichen oder tierischen Körpers darstellen.
